# EUROPEAN PATENT APPLICATION

(11) **EP 1 138 373 A1**
(43) Date of publication of application: **04.10.2001**
(21) Application number: 01201170.6
(22) Date of filing: 26.03.2001
(51) Int. Cl.: B01F 7/16, C12M 1/16

(54) **Solids fermentation reactor**

(30) Priority: 29.03.2000 NL 1014783
(71) Applicant: Hosokawa Micron B.V., 7005 BL Doetinchem (NL)
(72) Inventor: van der Wel, Peter Gerardus Josephus, 7006 TL Doetinchem (NL); Oostra, Jaap, 6706 AB Wageningen (NL); Rinzema, Arjen, 6708 SL Wageningen (NL); Timmer, Hendrik Roel, 6717 VB Ede (NL)
(74) Representative: Van Assen, Jan Willem Bernard

(57) **Abstract**

Solids fermentation reactor (SFR), comprising a lockable vertical conical mixer 1, preferably having a spherical lid 9 and at the lower side preferably a spherical valve 3.

According to one embodiment the mixer 1 contains a transport screw 5, according to another embodiment a mixing ribbon 8, which rotate both around their axes as well as along the wall.

The novel SFR reactor has the advantage that the fermentating reactor can be unloaded gradually, having a larger heat transmission with a homogeneous temperature distribution.

Furthermore, the fermentating reactors can be sterilized and it is possible to take sterile samples. It is also possible to scale up the concept in an easy way.

## Description

The invention relates to a reactor for executing solids fermentations (SFR).

From WO 94/18306 (EP 0.683.815) such a reactor is known, comprising:
- a lockable verticle mixing vessel;
- a verticle mixing member that during functioning can move close to the wall of the mixing vessel;
- an inlet for the matter to be fermented in a lid on the upper side of said mixing vessel;
- sterilization means;
- heating and/or cooling means;
- inocculation means including means for introducing a solution of feed stuffs;
- an outlet on the lower side of said mixing vessel, which outlet is intended for products which have been subjected to fermentation.

The fermentating reactor according to WO 94/18306 (EP 0.683.815) is a cylindrical mixing vessel which is provided at the lower side with a pivotable circular door or lid.

It is of course possible to cool the contents of the fermentating reactor in the fermentating reactor itself.

According to the invention the reactor for executing solid state fermentations (SFR) comprises a conical vertical mixer 1 having a mixing element which is moveable along the wall and an inlet 2 at the upper side and a outlet valve 3 at the lower side.

The application of a conical mixer has the following advantages:
. The conical vessel has a better evacuation as a result of the hopper shape.
. There is a relatively large heat exchange surface, there are no dead angles where material can remain.
. The combination of a conical vessel having a mixing element which is moveable along the wall, causes a homogenous temperature distribution and an optimal mixing.
. The conical shape of the wall relieves a part of the gravity on the lower part of the substrate, so that this material is crushed less easily.

The fermentating reactor can be unloaded more gradually.

Another advantage is that the fermentating reactors can be sterilized and also operated sterile, whereas sterile samples can be taken.

Furthermore, it is easily possible to scale up this design.

It is remarked, that the above mentioned conical mixer 1 can be provided with all auxilary means necessary for the method, such as for example looking glasses, temperature sensors, pressure sensors, sampler devices, inlet devices for steam and gasses, such as air, nitrogen or carbon dioxide, etc.

In connection with the use of a conical mixing vessel with solid state fermentation (SFR) there is also mentioned the DDR (East German) patent publication DD 260.837. Figure 2a thereof shows a mixer for solids fermentation (SFR), comprising a horizontal mixing vessel, which is double conical, having a horizontal mixing shaft with perpendicularly directed, stiff or elastic mixing or wiping strips.

In connection with DD 260.837 it is remarked, that this mixer has a large non-active volume. The large non-active volume takes a large volume, which is especially less advantageous when the contents of the fermentating reactor have to be cooled or sterilized.

According to an advantageous embodiment the conical mixer 1 is a conical vessel 4 having one or more transport screws 5 according to a descriptive line of the conical vessel, fastened to one or more radial arms 6 and rotateable around the shaft 7 thereof.

According to another advantageous embodiment the conical mixer 1 comprises a conical mixing vessel 4 with one or more mixing ribbons 8 along the inner surface of the mixing vessel 4, each fastened by arms 6 to the rotation shaft 7 through the centre of the mixing vessel 2.

The conical mixer 1 is preferentially provided with a substantially spherical lid 9.

A spherical lid has the advantage that there is more space for process auxiliaries, such as the inlet 2, and devices for the evacuation of gasses, etc.

According to a very advantageous embodiment the outlet valve 3 is a spherical valve.

A spherical valve has the advantage of being highly trouble free, well sealable and having good metering possibilities when unloading the fermentating reactor.

In connection with the above here follow some more elucidations of the reactor discussed, also named solids fermentation reactor (SFR) and the application to the fermentation process.

Finally two figures follow with a description of the preferential embodiment.

### The solids fermentating reactor

A device adapted for the solids fermentation process comprises:
- a cone shaped tank having a spherical lid
- the tank and the lid are provided with a double wall or a halve pipe spiral, so that the contents of the tank can be heated and/or cooled,
- the contents of the tank are mixed by means of a screw that can move along the wall or by a centrally driven ribbon shaped mixing member.
- at the lower side of the tank there is a spherical valve for the removal of the contents of the tank;
- with the exception of the described holes the contents of the tank are hermetically locked off, in order to preclude contamination of the contents and leaking out of material from the fermentating reactor;
- at the lower side of the tank there are inlet possibilities for steam and gasses, such as air, nitrogen or carbon dioxide;
- in the wall of the tank one or more temperature sensors have been mounted;
- a sampler can be mounted in the wall of the tank;
- in the lid there are means for :
- spraying in of cultures of micro organisms and/or liquids;
- the removal of gasses and vapours;
- measuring of temperature and pressure;
- filling the tank with substrate;
- inspecting and cleaning out respectively of the tank;
- one or more temperature sensors can be mounted centrally in the tank through the drive.

Typical sizes of the fermentating reactor vary from 5 litre for the laboratory embodiment to 10 to 20 m³ for industrial applications.

It is remarked, that the fermentating reactors can be sterilized and also operated sterile. It is possible to sample in a sterile way.

### The fermentation process

A process for the fermentation of solids can for example include:
1) the fermentating reactor is filled with the solid to be fermented;
2) the tank is filled and the contents are sterilized by a combination of heating of the wall and the injection of steam, so that the contents are held on a certain temperature for sufficient length;
3) small tanks can also be sterilized as a whole in an autoclave;
4) after the adjustment of the right temperature of the contents of the tank, the culture of the micro organism is sprayed on the substrate;
5) during the fermentation process the temperature, humidity, acidity and oxigen content of the contents of the tank are checked and adjusted, mostly through the inlet of gasses and vapour at the lower side of the tank and the removal of waste gasses through the upper side;
6) during the fermentation process the contents of the tank can be mixed in order to homogenize the temperature, simplify the introduction and removal of gasses and vapour and to preclude that the contents of the tank grow together. This mixing usually takes place slowly and sometimes also discontinually;
7) the progress of the fermentation process is followed by determination of the removed gasses and vapours, visually through the inspection windows and/or through samples that are removed sterily from the tank through the sampler in the wall of the tank;
8) after the end of the fermentation process the produced material is unloaded through the unloading valve at the lower side.

In connection with the mixing mentioned under 6) the following is still remarked.

With respect to the above mentioned East German patent publication DD 260.837, it is mentioned by partly the same inventors in the later German patent publication DE DD 294.043, that a certain solid fermentation transformation on reduced weed grain does not run well (viz. page 1 of DE DD 294.043 the penultimate paragraph).

The device, which has apparantly been applied, is one of the devices according to figures 1, 2a and 2b of the East German patent publication DD 260.837. All these devices are horizontal mixing vessels with a horizontal mixing shaft.

In the DE DD 294.043 there is now remarked, that the sticking together of the grains of the substrate disturbs the conversion. There is namely a tendency of sticking together between fungusmycelium and substrate.

In order to obviate this problem according to DE DD 294.043 a porous substrate is applied, on which inocculate and feedstuff solutions are added and it is placed in a 500 ml Erlenmeyer container, kept 5 days at 25EC and shaken loose every 24 hours (see p. 2 and 3 of DE DD 294.043).

According to the invention it has now appeared, that the use of the above mentioned conical mixer gives excellent possibilities to obviate the sticking together of certain substrates, and that therefore by a good control of the process parameters solid fermentations can be executed better than before.

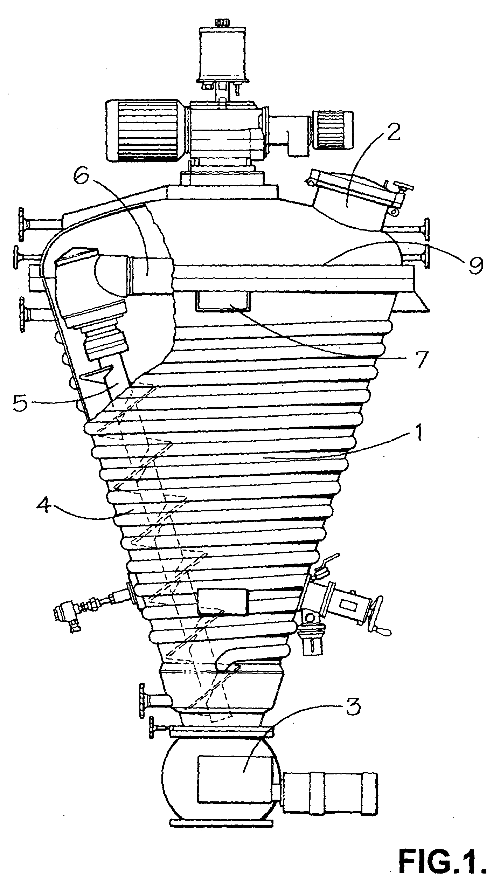

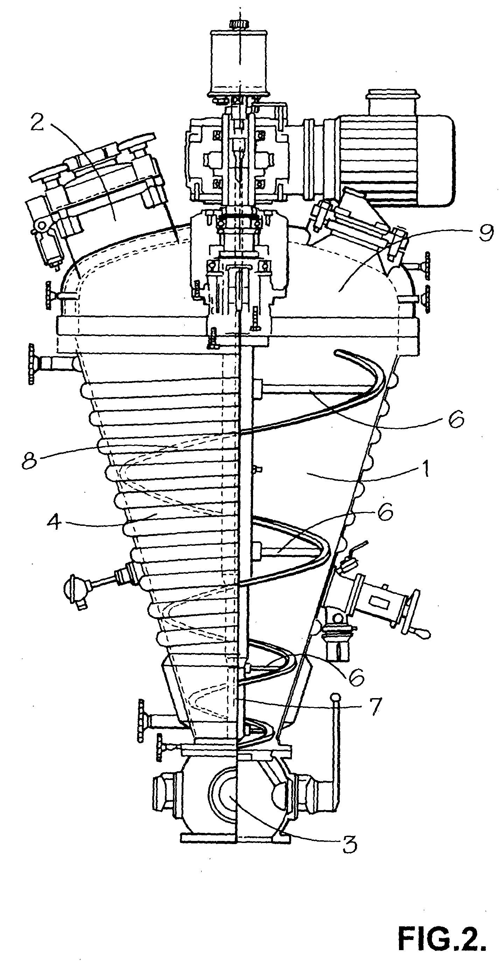

In the following description of the pertaining figures 1 and 2 two preferential embodiments are described of a solids fermentation reactor (SFR) according to the invention.

Figure 1 is a side view of a reactor of which a part of the sidewall has been removed, in which reactor a transportation screw suspended on an arm is used as mixing means.

Figure 2 is also a side view of the reactor of which the right side of a part of the wall has been removed, in which a mixing ribbon, which is moveable along the inner surface of the mixing vessel, is used as mixing member.

In both descriptions the same members have been provided with the same reference numerals.

In figures 1 and 2 the numerals 1, 2, 3 and 4 respectively denote a conical mixer 1, an inlet 2, an outlet valve 3 and a conical mixing vessel 4.

Furthermore in figures 1 and 2 the numerals 6, 7 and 9 respectively denote the arms 6, a rotation shaft 7 around the centre line of the conical mixing vessel 4 and the lid' 9 of this mixing vessel.

In both figures 1 and 2 the numerals 9 and 3 respectively denote a spherical valve 3 and a spherical lid 9.

In figure 1 a transportation screw 5 has been mounted by means of an arm 6 to a rotation shaft 7 through the centre line of the mixing vessel 4.

In figure 2 a ribbon shaped mixing member 8 is mounted by means of a number of arms 6 to a rotation shaft 7 through the centre line of the mixing vessel 4.

## Claims

1. Reactor for solids fermentations (SFR), comprising:
- a closeable vertical conical mixing vessel;
- a vertical mixing member, that during the operation can move close to the wall of the mixing vessel;
- an inlet for products to be fermented in a lid at the upper side of said mixing vessel;
- sterilization means;
- heating and/or cooling means;
- inocculation means, including means for the introduction of feedstuff solutions;
- an outlet for products, after these have been subjected to fermentation, at the lower side of said mixing vessel;
**characterized in that** the reactor is a conical vertical mixer (1) having an inlet (2) at the upper side and an outlet valve (3) at the lower side.

2. Reactor according to claim 1, **characterized in that**, the conical mixer (1) being a conical tank (4) having one or more transportations screws (5) according to a line extending close to the conical tank (1), fastened to one or more arms (6) at the upper side of said conical vessel (4) and rotatable around the shaft (7) thereof.

3. Reactor according to claim 1, **characterized in that**, the conical mixer (1) comprises a conical mixing tank (4) having one or more mixing ribbons (8) along the inner surface of the mixing tank (4), in which each mixing ribbon (8) is rotatably fastened by arms (6) around the rotation shaft (7) through the centre line of the mixing tank (4).

4. Reactor according to each of the above claims, **characterized in that**, the conical tank (1) is provided with a substantially spherical lid (9).

5. Reactor according to each of the above claims, **characterized in that**, the outlet valve (3) being a spherical valve.
